(19) **Europäisches Patentamt**
**European Patent Office**
**Office européen des brevets**

(11) **EP 2 351 829 A1**

(12) **EUROPEAN PATENT APPLICATION**
published in accordance with Art. 153(4) EPC

(43) Date of publication:
**03.08.2011 Bulletin 2011/31**

(51) Int Cl.:
*C12N 1/16* (2006.01)  *A23L 1/221* (2006.01)
*A23L 1/28* (2006.01)  *A23L 1/39* (2006.01)

(21) Application number: **09827339.4**

(22) Date of filing: **17.11.2009**

(86) International application number:
**PCT/JP2009/006165**

(87) International publication number:
**WO 2010/058558 (27.05.2010 Gazette 2010/21)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**RS**

(30) Priority: **18.11.2008 JP 2008294643**

(71) Applicant: **Asahi Breweries, Ltd.**
**Sumida-ku**
**Tokyo 130-8602 (JP)**

(72) Inventors:
• **SHIBUYA, Ichiro**
  **Moriya-shi**
  **Ibaraki 302-0106 (JP)**
• **ODANI, Tetsuji**
  **Moriya-shi**
  **Ibaraki 302-0106 (JP)**

(74) Representative: **Desaix, Anne et al**
**Ernest Gutmann - Yves Plasseraud S.A.S.**
**3, rue Auber**
**75009 Paris (FR)**

(54) **METHOD FOR PRODUCING ALANINE-RICH YEAST**

(57) An object of the present invention is to provide a method for producing an alanine-rich yeast which contains alanine at a high concentration. The present invention provides a method for producing an alanine-rich yeast, which includes liquid-culturing a yeast in a stationary phase of proliferation under the condition that the pH of a liquid medium is equal to or higher than 7.5 and lower than 11; a method for producing an alanine-rich yeast, which includes adjusting the pH of a liquid medium of the yeast in a stationary phase of proliferation to be equal to or higher than 7.5 and lower than 11, and then culturing the yeast in the above pH range; a method of producing an alanine-rich yeast, wherein the yeast is Saccharomyces cerevisiae or Candida utilis; an alanine-rich yeast obtained by any one of the above methods for producing an alanine-rich yeast; an alanine-rich yeast extract extracted from the above alanine-rich yeast; a seasoning composition including the above alanine-rich yeast extract; and an alanine-containing food and drink, including the above alanine-rich yeast, the above alanine-rich yeast extract or the above seasoning composition.

FIG. 1

**Description**

[Technical Field]

The present invention relates to a method for producing an alanine-rich yeast, an alanine-rich yeast, an alanine-rich yeast extract, a seasoning composition, and an alanine-rich yeast extract-containing food and drink.

[Background Art]

Currently, mainly in advanced countries such as Europe, USA, and Japan, natural- and health-conscious natural seasonings which do not use additives are required all over the world. Under such circumstances, in the yeast extract industry, high-value added extracts which have a high amount of taste-active amino acids such as glutamic acid and nucleic acids, and also have enhanced "deliciousness (umami)" have been developed.
As for glutamic acid, sodium glutamate has hitherto been spread as a chemical seasoning. However, in recent years, a yeast extract containing abundantly not only glutamic acid but also other amino acids is favored from the viewpoint of not only taste quality but also from the viewpoint of health.
For example, Patent Literature 1 describes a yeast extract characterized in that the amount of free amino acid is 25% by weight or more, and also the total amount of nucleic acid-based taste-active components is 2% by weight or more.
Also, Patent Literature 2 describes a yeast extract composition derived from an yeast belonging to the genus Candida tropicalis, Candida lipolytica or Candida utilis, in which the amount of a total free amino acid in a yeast extract is 3.0% or more, the amount of alanine in the amount of total free amino acid is 10% or more, the amount of glutamic acid is 25% or more, and the amount of histidine is 10% or more.

[Citation List]

[Patent Literature]

[Patent literature 1]
Japanese Unexamined Patent Application, First Publication No. 2007-49989
[Patent literature 2]
Japanese Patent No. 3,519,572

[Summary of Invention]

[Technical Problem]

However, in conventional methods, in order to include a sufficient amount of a free amino acid in the case of producing various extracts using a vegetable or animal protein, operation becomes complicated in many cases, for example, it is necessary to carry out decomposition treatments such as hydrolyzed vegetable protein (HVP) and hydrolyzed animal protein (HAP). In addition, there has been required a method for producing a yeast extract containing an amino acid or the like in a higher concentration than that of a yeast extract which is currently commercially available.
Particularly, development of a method capable of producing a yeast extract having a higher content of alanine in the yeast extract in a simple and easy manner has been required.
This is because while many amino acids may exhibit astringency, it can be expected that astringency of the yeast extract can be suppressed by controlling the amount of alanine in the yeast extract.
Regarding Patent Literature 1, operations such as use of an enzyme are complicated.
In addition, regarding Patent Literature 2, in addition to a complicated operation such as use of an enzyme, a yeast subjected to a mutation treatment is used, resulting in poor safety, preference and the like as a food.
Under these circumstances, the present invention has been made and an object of the present invention is to provide a method for producing an alanine-rich yeast containing alanine in a higher concentration than that of a conventional yeast, an alanine-rich yeast, an alanine-rich yeast extract, a seasoning composition, and an alanine-containing food and drink.

[Solution to Problem]

The present inventors have intensively studied the situation so as to achieve the above object and found that the amount of alanine in a yeast increases by increasing the pH of a culture fluid to a specific pH (shifting to an alkaline region) during culturing of the yeast in a stationary phase. They have also found that a yeast extract having a high amount of alanine can be produced by producing a yeast extract using this yeast, and thus the present invention has been completed.

The present invention employs the following constitutions.

[1] A method for producing an alanine-rich yeast, which includes liquid-culturing a yeast in a stationary phase of proliferation under the condition that the pH of a liquid medium is 7.5 or higher and lower than 11.

[2] The method for producing an alanine-rich yeast according to [1], wherein the liquid-culturing includes adjusting the pH of a liquid medium of the yeast in a stationary phase of proliferation to 7.5 or higher and lower than 11, and culturing the yeast in the above pH range.

[3] The method for producing an alanine-rich yeast according to [1] or [2], wherein the yeast is Saccharomyces cerevisiae or Candida utilis.

[4] An alanine-rich yeast obtained by the method for producing an alanine-rich yeast according to any one of [1] to [3].

[5] An alanine-rich yeast extract extracted from the alanine-rich yeast according to [4].

[6] A seasoning composition including the alanine-rich yeast extract according to [5].

[7] An alanine-containing food and drink, including the alanine-rich yeast according to [4], the alanine-rich yeast extract according to [5] or the seasoning composition according to [6].

[Advantageous Effects of Invention]

According to the method for producing an alanine-rich yeast of the present invention, an alanine-rich yeast having a remarkably increased amount of alanine can be produced in a simple and easy manner only by shifting the pH of a liquid medium of the yeast in a stationary phase to an alkali region.

By performing an operation of extraction from the alanine-rich yeast of the present invention, an alanine-rich yeast extract containing alanine at a high concentration is obtained.

[Brief Description of Drawings]

Fig 1 is a graph showing a curve of the increase in a cell count versus the culture time in Example 1.

Fig. 2 is a graph showing a curve of the increase in weight of dry yeast cells versus the culture time in Example 1.

Fig. 3 is a graph showing a change in the pH of a liquid medium versus the culture time in Example 1.

[Description of Embodiments]

The method for producing an alanine-rich yeast of the present invention includes liquid-culturing a yeast in a stationary phase of proliferation under the condition that the pH of a liquid medium is 7.5 or higher and lower than 11. Embodiments of the present invention will be described in detail below.

The yeast may be unicellular fungi and specific examples thereof include fungi of the genus Saccharomyces, fungi of the genus Shizosaccharomyces, fungi of the genus Pichia, fungi of the genus Candida, fungi of the genus Kluyveromyces, fungi of the genus Williopsis, fungi of the genus Debaryomyces, fungi of the genus Galactomyces, fungi of the genus Torulaspora, fungi of the genus Rhodotorula, fungi of the genus Yarrowia, fungi of the genus Zygosaccharomyces and the like.

Among these yeasts, from the viewpoint of edibility, Candida tropicalis, Candida lypolitica, Candida utilis, Candida sake, Saccharomyces cerevisiae and the like are preferable, and commonly used Saccharomyces cerevisiae and Candida utilis are more preferable.

In order to carry out the present invention, after the yeast is cultured in a liquid medium containing a carbon source, a nitrogen source, an inorganic salt or the like until a stationary phase, the yeast may be liquid-cultured under the condition that the pH of a liquid medium of the yeast in a stationary phase of proliferation is 7.5 or higher and lower than 11.

A medium composition of these bacterial strains is not particularly limited, and compositions which are utilized in a conventional method can be used. For example, as the carbon source, one or two or more kinds selected from the group consisting of glucose, sucrose, acetic acid, ethanol, molasses, a sulfite pulp waste solution and the like, which are utilized in conventional culturing of microorganisms, are used. As the nitrogen source, one or two or more kinds selected from the group consisting of urea, ammonia, inorganic salts such as ammonium sulfate, ammonium chloride or ammonium phosphate, and nitrogen-containing organic substances such as corn steep liquor (CSL), casein, yeast extract or peptone, are used. Furthermore, a phosphate component, a potassium component, and a magnesium component may be added to the medium, and conventional industrial raw materials such as calcium superphosphate, ammonium phosphate, potassium chloride, potassium hydroxide, magnesium sulfate and magnesium hydrochloride may be used. In addition, inorganic salts such as zinc, copper, manganese and iron ions may be used. In addition, vitamins, nucleic acid-associated substances and the like may be added.

As a culturing form, any of batch culturing, semi-batch culturing and continuous culturing may be used. However, semi-batch culturing or continuous culturing is industrially employed.

The culturing condition in a logarithmic proliferation phase or the culturing condition before pH adjustment may be according to the general condition for culturing yeast and, for example, the temperature is from 20 to 40°C, and is preferably from 25 to 35°C, and the pH is from 3.5 to 7.5, and is particularly desirably from 4.0 to 6.0. In addition, the aerobic condition is preferred.

It is preferable to perform culturing while aeration and stirring. The amount of aeration and stirring condition can be appropriately determined by taking account of the volume and the time of culturing, and the initial concentration of a fungus. For example, culturing can be performed while aeration of about 0.2 to 2 volume per volume per minute (V.V.M.) and stirring at about 50 to 800 rpm.

The method of liquid-culturing under the condition that the pH of a liquid medium of the yeast in a stationary phase of proliferation is 7.5 or higher and lower than 11 is not particularly limited. For example, the pH of a liquid medium may be adjusted to be equal to or higher than 7.5 and lower than 11 when yeast culturing has entered a stationary phase. Alternatively, a liquid medium may be shifted by adding urea to a medium in advance such that the pH naturally becomes equal to or higher than 7.5 and lower than 11 with a lapse of the culture time.

The amount of urea or the like to be added to the medium is not particularly limited, and is preferably from about 0.5 to 5% relative to the medium, although it depends on the cell concentration of the yeast to be cultured.

The method of adjusting the pH of a liquid medium to be equal to or higher than 7.5 and lower than 11 when the cultured yeast has entered a stationary phase is not particularly limited. For example, an alkaline component is appropriately added, and the pH of a liquid medium may be adjusted to be higher than 7.5 and lower than 11, and preferably to be higher than 7.5 and lower than 10.

The adjustment of the pH may be performed as long as yeast is in a stationary phase, and it is preferable to perform pH adjustment immediately after yeast has entered a stationary phase. This is because not only can the concentration of alanine in the yeast be sufficiently enhanced, but also the require time until completion of all steps can be shortened. When the pH of the liquid medium of the yeast in a logarithmic proliferation phase is adjusted to be equal to or higher than 7.5 and lower than 11, proliferation of the yeast is suppressed, and thus the free alanine content is not increased, which is unfavorable.

Examples of the alkaline component include, but are not limited to, inorganic alkalis such as $NH_4OH$ (aqueous ammonia), ammonia gas, sodium hydroxide, potassium hydroxide, calcium hydroxide, and magnesium hydroxide; alkaline bases such as sodium carbonate, and potassium carbonate; organic alkalis such as urea; and the like.

Among these components, aqueous ammonia, ammonia gas and urea are preferable.

The temperature and other conditions when the yeast in a stationary phase is cultured in a liquid medium having a pH of higher than 7.5 and lower than 11 may be according to the common condition for culturing yeast, and for example, the temperature is from 20 to 40°C, and is preferably from 25 to 35°C.

There is a tendency that the amount of alanine in the yeast after the pH has shifted to higher than 7.5 and lower than 11 reaches a peak and, thereafter, the amount decreases with a lapse of a culture time. In addition, this depends on the conditions such as the cell concentration of the yeast to be cultured, the pH and the temperature. This is surmised that when excessively cultured for a long term under alkaline conditions, an influence of an alkali on the yeast becomes too great. Therefore, in the present invention, an optimal culture time can be appropriately selected for every culturing condition, particularly, for every pH after alkali shift. By preparing a yeast extract using the yeast at a peak, an alanine-rich yeast extract having a very high amount of alanine of 2.0% by weight or more, and preferably of 2.0 to 12.0% by weight on a dry weight basis, is obtained.

In the present invention, "the amount of alanine on dry weight basis of the yeast extract" means the ratio (% by weight) of alanine contained in solid parts obtained by drying the yeast extract.

As the method of measuring the amount of alanine, for example, it can be measured by the AccQ-Tag Ultra labeling method using the Acquity UPLC analyzing apparatus manufactured by Waters (USA). A calibration curve may be made, for example, using an amino acid mixed standard solution H type (manufactured by Wako Pure Chemical Industries, Ltd.).

The amount can also be measured using an amino acid automatic analyzing apparatus manufactured by JEOL Ltd.,

Model JLC-500/V, but the method is not particularly limited.

According to the method of the present invention, a yeast abundantly containing alanine, and particularly containing free alanine in cells can be produced. For this reason, by extraction of a yeast extract from the yeast, a yeast extract abundantly containing free alanine as a satisfactory taste-active component can be obtained in a simple and easy manner. Since the thus obtained yeast extract has a high amount of free alanine, astringency peculiar to an amino acid is suppressed, and the yeast extract is more satisfactory yeast extract.

The present invention can produce alanine-rich yeast by a simple step of only performing alkali shift of liquid medium. As described above, it is not necessary to use a particularly special medium, but the yeast can be produced using an inexpensive raw material such as ammonia.

According to the method of the present invention, an alanine-rich yeast containing alanine in a high concentration within yeast cells is obtained. However, a fraction containing alanine may be obtained from the alanine-rich yeast. As the method of fractionating a fraction containing alanine from the alanine-rich yeast, any method may be used as long as it is a method which is usually performed.

An alanine-rich yeast extract can be produced from the alanine-rich yeast cultured by the above method. As a method of producing the alanine-rich yeast extract, any method may be used as long as it is a method which is usually performed, and a self-digesting method, an enzyme degrading method, an acid degrading method, an alkali extracting method, a hot water extracting method and the like are employed. Commonly, it is considered that alanine in a yeast extract obtained only by the hot water extracting method is free alanine in an approximately all amount, unlike the yeast extract obtained by an enzyme reacting method such as the self-digesting method.

The alanine-rich yeast of the present invention contains a large amount of a free amino acid and, therefore, even when a yeast extract is simply extracted only by a hot water treatment, a yeast extract having satisfactory taste is obtained.

Heretofore, in order to enhance the amount of a taste-active amino acid such as free alanine, a hydrolysis treatment using an acid or an alkali has been commonly performed using a vegetable or animal protein. However, a hydrolysate of a protein has a problem that it contains MCP (chloropropanols) which is suspected to be carcinogenic.

To the contrary, since the alanine-rich yeast produced by the method of the present invention has originally a high free amino acid content, a yeast extract having a sufficiently high amount of free alanine can be prepared without a decomposition treatment with an acid or an alkali, or enzyme treatment, after the yeast is extracted by the hot water method or the like. That is, by using the alanine-rich yeast of the present invention, a yeast extract excellent in both of taste-active property and safety can be produced in a simple and easy manner.

Therefore, the yeast extract obtained by the present invention has a very high taste-active property and, by being used in foods and drinks, it is possible to produce foods and drinks having a deep taste and richness.

Furthermore, by formulating the alanine-rich yeast extract of the present invention into powders, an alanine-rich yeast extract powder is obtained and by appropriately selecting a yeast fungus, a yeast extract powder containing 5.0% by weight or more on dry weight basis of alanine is obtained.

Dry yeast cells may be prepared from the alanine-rich yeast cultured by the above method. As the method of preparing the dry yeast cell, any method may be used as long as it is a conventional method. However, a lyophilization method, a spray drying method, a drum drying method and the like are employed.

The alanine-rich yeast, dry yeast cells of the yeast, a yeast extract prepared from the yeast, and the yeast extract powder of the present invention may be formulated into a seasoning composition. The seasoning composition may consist only of the yeast extract of the present invention, or may contain other components such as a stabilizer or a preservative, in addition to the yeast extract or the like of the present invention. The seasoning composition can be appropriately used in various foods and drinks, similar to other seasoning compositions.

Furthermore, the present invention relates to food and drink containing the alanine-rich yeast obtained by the above method, or the alanine-rich yeast extract extracted from the alanine-rich yeast. By including the alanine-rich yeast or the like of the present invention, foods and drinks containing alanine at a high concentration can be effectively produced.

These foods and drinks may be any food and drink as long as they are food and drink to which dry yeast, a yeast extract, and a seasoning composition containing them can be added, and examples thereof include alcoholic drinks, refreshing drinks, fermented foods, seasonings, soups, breads, confectionaries and the like.

In order to produce the foods and drinks of the present invention, a preparation obtained from the above alanine-rich yeast, or a fraction of the alanine-rich yeast may be added in the production process of foods and drinks. In addition, as a raw material, the alanine-rich yeast may be used as it is.

The present invention will be described in more detail by way of Examples, but the present invention is not limited to the following Examples.

Example 1

Saccharomyces cerevisiae AB9813 strain was cultured, and extract extraction from a yeast culture, and alanine analysis were performed, by the methods shown in the following <1> to <8>.

<1> Pre-culturing

Two media consisting of the following composition were prepared (volume of 350 mL, 2 L baffled Erlenmeyer flask) .

| (Medium composition) | |
|---|---|
| Molasses | 8% |
| Urea | 0.6% |
| $(NH_4)_2SO_4$, | 0.16% (Ammonium sulfate) and |
| $(NH_4)_2HPO_4$ | 0.08% (diammonium hydrogen phosphate) |

(Preparation method)

(1) Molasses (sugar degree 36%, 167 ml) was diluted to 750 ml with milli-Q water, and then each 350 ml was dispensed into a 2 L baffled Erlenmeyer flask.
(2) Autoclaving (121°C, 15 min) was performed.
(3) Upon use, a 1/50 amount (each 7 mL) of a nitrogen component mixed solution ($\times$100) was added sterilely to a medium of only molasses.

| (Culturing conditions) | |
|---|---|
| Culture temperature | 30°C |
| Shaking | 160 rpm (rotary) |
| Culture time | 24 hours |
| (Inoculating fungus amount: 300 mL) | |

<2> Main culturing

A medium consisting of following composition was prepared at a volume of 2,000 mL (set at 3 L at the completion of semi-batching).

| (Medium composition) | |
|---|---|
| Ammonium chloride | 0.18% (in terms of 3 L at the completion of semi-batching) 5.3 g |
| $(NH_4)_2HPO_4$ | 0.04% (diammonium hydrogen phosphate, in terms of the completion of semi-batching) 1.2 g |

Subsequently, culturing was performed under the following conditions.

| (Culturing condition) | |
|---|---|
| Culture temperature | 30°C |
| Aeration | 3 L/min |
| Stirring | 600 rpm |
| pH control | Lower limit control pH 5.0 |
| (with 10% aqueous ammonia), no upper limit control | |
| Anti-foaming agent | Adecanate stock |
| Semi-batching medium | Molasses (sugar degree 36%), |
| volume 800 mL (with 1 L Medium bottle, final 8%) | |

<3> pH shift

Then, immediately after the cultured yeast had entered a stationary phase, the pH of the culture fluid was shifted to an alkaline region (hereinafter, referred to as pH shift) with an aqueous $NH_4OH$ solution (10%) (target pH: 9.0), followed by further culturing of the yeast. After 48 hours from initiation of main culturing, the culturing was completed.

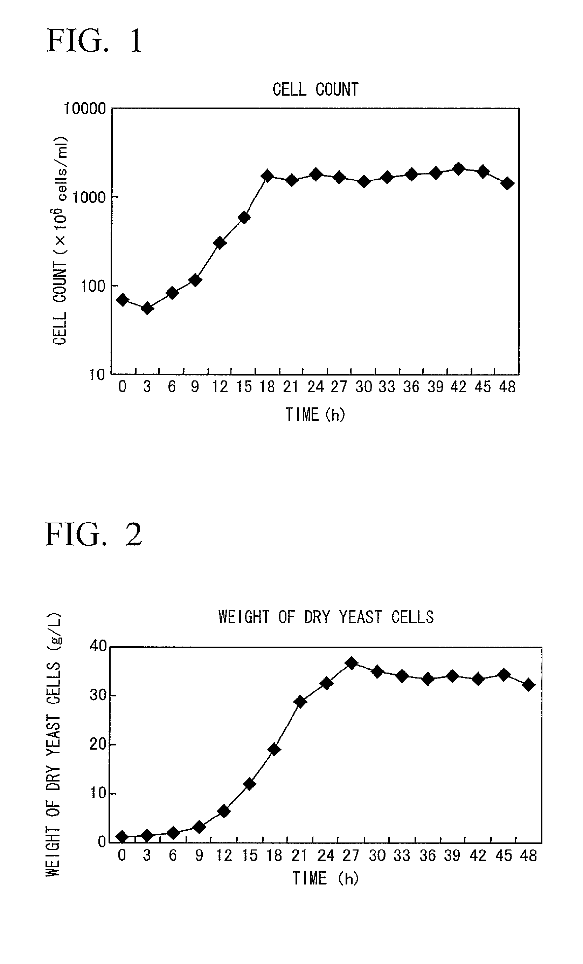

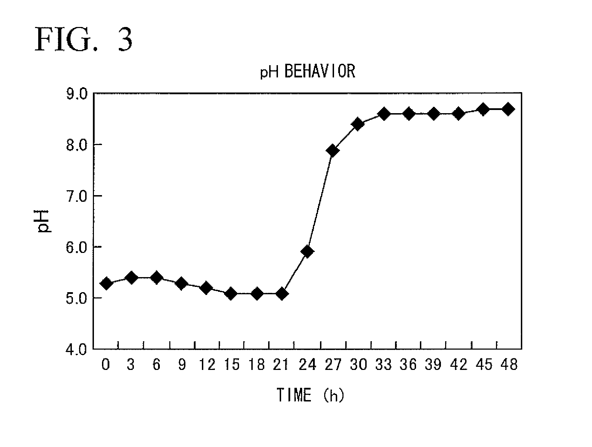

Fig. 1 is a graph showing a curve of the increase in cell count versus the culture time. Fig. 2 is a graph showing a curve of the increase in the weight of dry yeast cells versus the culture time. Fig. 3 is a graph showing the change in pH of a culture fluid versus the culture time.
As shown in Fig. 1, it was confirmed that the increase in cell count ($\times 10^6$ cells/ml) reached the stationary state after 18 hours from culturing, and yeast entered a stationary phase of proliferation. In addition, the weight of dry yeast cells (g/L) also entered an approximately stationary state after 24 hours from culturing, and a stationary phase of proliferation was confirmed. The pH of the culture fluid was measured and, as a result, as shown in Fig. 3, the pH was shifted to an alkali region (7.5 or higher and lower than 11), after the yeast had entered a stationary phase of proliferation.

<4> Cell collecting method

(1) The culture fluid in which yeast had been main-cultured was transferred to a 50 ml plastic centrifugation tube (Falcon 2070), and centrifugation (3,000 g, 20°C, 5 min, HP-26) was performed.
(2) The supernatant was discarded and pellets were suspended in 20 ml of milli-Q water, and then centrifugation (3,000 g, 20°C, 5 min, HP26) was performed. This operation was repeated twice.
(3) The supernatant was discarded, and pellets were suspended in 20 ml of milli-Q water.

<5> Measurement of the weight of dry yeast cells

Into an aluminum dish (diameter 5 cm) which had been weighed in advance was taken 2 ml of a yeast suspension, and this was dried at 105°C for 4 hours.
A weight after drying (weight after drying yeast) was measured, and the weight of solid parts (weight of dry yeast cells, unit g/L) was calculated by the following equation (1).

$$\text{Weight after drying} - \text{weight of aluminum dish} = \text{weight of dry yeast cells} \qquad (1)$$

<6> Preparation of extract solution by hot water extracting method

(1) The remaining yeast suspension (about 18 ml) was centrifuged (3,000 g, 20°C, 5 min, HP-26).
(2) The remaining suspension (1.5 ml) was transferred to an Eppendorf tube, and the tube was transferred to a block heater and then heated at 80°C for 30 minutes (conversion into extract). Alternatively, it may be excessively heated in a warm bath at 100°C for 10 minutes (conversion into extract).
(3) Thereafter, the supernatant (extract solution) was separated by centrifugation (6,000 g, 4°C, 5 min).

<7> Method for measurement of decomposition rate

The extract solution (500 μl) was taken into an aluminum dish and dried at 105°C for 4 hours. Thereafter, an extract weight ratio (w/w) was calculated from a dry weight before conversion into an extract.

<8> Method for measurement of the amount of alanine

The amount of alanine per weight of dry yeast cells of each of the yeast before pH shift and yeast after pH shift was measured. Specifically, the amount of alanine was measured by the AccQ-Tag Ultra labeling method using the Acquity UPLC analyzing apparatus manufactured by Waters (USA). In the measuring method, free alanine in a sample can be selectively determined. A calibration curve was made using an amino acid mixed standard solution (type H) (manufactured by Wako Pure Chemical Industries Ltd.). The measurement results are shown in Table 1.

Table 1

|  | 30°C, target pH: 9 | 30°C, target pH: 9 |
| --- | --- | --- |
|  | Before shift | After shift |
| Weight of dry yeast cells (g/L) | 87.3 | 87.9 |
| Alanine (% by weight in dry yeast cells) | 1.6 | 2.4 |

As is apparent from the above results, the amount of alanine in yeast is mostly increased at about 1.5-fold immediately after a pH shift and, thereafter, an amount of alanine higher than that before the pH shift can be maintained. As is apparent from the above result, alanine in yeast is increased by further culturing yeast by adjusting the pH to be equal to or higher than 7.5 and less than 11 after a stationary phase.

As described above, in the present Example, free analine in the sample is measured and, therefore, the amount therof shown in Table 1 is the amount of free alanine.

Example 2

Then, both the amount of alanine per weight of dry cells of the yeast prepared in the same manner as in Example 1 (pH 9.0), and the amount of alanine on dry weight basis of a yeast extract prepared from this yeast were measured. The measurement results are shown in Table 2.

Table 2

| Target pH |  | Amount of alanine (% by weight of dry yeast cells) | Amount of alanine (% by weight of dry yeast cells) |
| --- | --- | --- | --- |
| 9.0 | Before pH shift | 1.4 | 5.7 |
|  | After pH shift | 2.2 | 8.9 |

As shown in Table 2, the amount of alanine (% by weight) in the dry fungal cell weight was increased from 1.4% by weight to 2.2% by weight by a pH shift. Also in a yeast extract prepared from these yeasts, the amount of alanine (% by weight) on a dry weight basis was increased from 5.7% by weight to 8.9% by weight, similarly. That is, it was confirmed that a yeast extract having a high amount of alanine is obtained by preparing a yeast extract from alanine-rich yeast produced using the method of the present invention.

Example 3

Subsequently, regarding a yeast extract produced from yeast prepared in the same manner as in Example 1 (pH 9.0), and commercially available yeast extracts (Comparative Examples 1 to 8), the amount of alanine per extract dry weight was measured, followed by comparison. In addition, alanine was measured in the same manner as in Example 1. The amount of alanine (% by weight) on a dry weight basis of respective yeast extracts, which were obtained as a result of measurement, are shown in Table 3.

Table 3

| Samples | Ex. 3 | Comp. Ex. 1 | Comp. Ex. 2 | Comp. Ex. 3 | Comp. Ex. 4 |
|---|---|---|---|---|---|
| Amount in extract | | Product of company A | Product of company B | Product of company C | Product of company D |
| Alanine (% by weight) | 8.9 | 0.9 | 0.0 | 1.8 | 1.4 |

| Samples | Comp. Ex. 5 | Comp. Ex. 6 | Comp. Ex. 7 | Comp. Ex. 8 |
|---|---|---|---|---|
| Amount in extract | Product of company E | Product of company F | Product of company G | Product of company H |
| Alanine (% by weight) | 0.8 | 0.3 | 0.9 | 0.5 |

As is apparent from the results, the yeast extract of the present invention contains a remarkably large amount of alanine, which is an amino acid having a taste-active property, and it was suggested that this yeast extract is preferable as a seasoning.

Example 4

Furthermore, a miso soup and a consomme soup were made using a yeast extract powder (derived from Saccharomyces cerevisiae) obtained by formulating, a yeast extract produced from the yeast prepared in the same manner as in Example 1 (pH 9.0), into a powder. The amount of the yeast extract blended with the miso soup or the consomme soup is 0.2%.
As Comparative Example, using Meast Powder N (manufactured by ASAHI FOOD and HEALTHCARE CO., LTD.), a miso soup and a consomme soup were prepared similarly, sensory evaluation was performed by the following method.

(Evaluation method)

A comparative sensory test was performed using a blind two points comparison by 10 professional panelists. As a 2 pair comparative test, a t-test was performed.

(Evaluation criteria)

With respect to three items, salty taste (salt reduction effect), deliciousness and richness, a five-rank evaluation was performed, assuming that a miso soup as a standard or a consomme soup as a standard is 0.

"Strong" = +2,
"Slightly strong" = +1,
"Neither" = 0
"Slightly weak" = -1,
"Weak" = -2.

The results of the miso soup are shown in Table 4, and the results of the consomme soup are shown in Table 5.
Table 4

| Samples | Items | Panels | | | | | | | | | | Average | Standard deviation |
|---|---|---|---|---|---|---|---|---|---|---|---|---|---|
| | | A | B | C | D | E | F | G | H | I | J | | |
| Examples | Salty taste | -1 | 1 | 1 | -1 | -1 | 1 | 0 | 0 | 0 | 1 | 0.10 | 0.88 |
| | Deliciousness | 0 | 0 | 1 | 2 | -1 | 0 | 1 | 1 | 1 | -1 | 0.40 | 0.97 |
| | Richness | 0 | 2 | 0 | 1 | 0 | 0 | 1 | 2 | 1 | 1 | 0.80 | 0.79 |
| Meast Powder N (Comparative Example) | Salty taste | 0 | 0 | 1 | -2 | 0 | -1 | 0 | 0 | -1 | 0 | -0.30 | 0.82 |
| | Deliciousness | 1 | 0 | 0 | 1 | -1 | -1 | 1 | 0 | -1 | 0 | 0.00 | 0.82 |
| | Richness | 1 | 2 | 0 | 0 | -1 | 0 | 0 | 0 | 0 | 0 | 0.20 | 0.79 |
| t-test for paired data<br>Salty taste p = 0.11<br>Deliciousness p = 0.11<br>Richness p = 0.03* | | | | | | | | | | | | | |

Table 5

| Samples | Items | Panels | | | | | | | | | | Average | Standard deviation |
|---|---|---|---|---|---|---|---|---|---|---|---|---|---|
| | | A | B | C | D | E | F | G | H | I | J | | |
| Examples | Salty taste | 1 | 1 | 1 | 1 | 0 | -1 | 1 | 0 | 0 | 1 | 0.50 | 0.71 |
| | Deliciousness | 1 | 0 | 1 | 2 | 1 | -1 | 2 | 2 | 0 | 1 | 0.90 | 0.99 |
| | Richness | 1 | 0 | 0 | 2 | 1 | 0 | 0 | 0 | 0 | 1 | 0.50 | 0.71 |
| Meast Powder N (Comparative Example) | Salty taste | 0 | -1 | 0 | -1 | 1 | 1 | 0 | 0 | 1 | -1 | 0.00 | 0.82 |
| | Deliciousness | 1 | -1 | 0 | -1 | 0 | 1 | 1 | 0 | 0 | -1 | 0.00 | 0.82 |
| | Richness | 1 | -1 | 1 | 1 | 1 | 1 | 0 | 0 | 1 | -1 | 0.40 | 0.84 |
| t-test for paired data<br>Salty taste p = 0.15<br>Deliciousness p = 0.03*<br>Richness p = 0.38 | | | | | | | | | | | | | |

As is apparent from the results of Table 4, in the miso soup, there was a difference in the average of salty taste and deliciousness, and there was a significant difference in richness. From the results of Table 5, in the consomme soup, there was a difference in the average of salty taste and richness, and there was a significant difference in deliciousness. It is considered that this is because the yeast extract of the present invention has a significantly higher amount of alanine compared with a conventional yeast extract.

Example 5

In the same manner as in Example 1, except that the yeast to be cultured is Saccharomyces cerevisiae ABS1 strain, a yeast was cultured, and extraction of an extract from a yeast culture fluid and alanine analysis were performed. Measured values of the amount of alanine before and after the pH shift are shown in Table 5.

Example 6

In the same manner as in Example 1, except that the yeast to be cultured is Saccharomyces cerevisiae ABS2 strain, a yeast was cultured, and extraction of an extract from an yeast culture fluid and alanine analysis were performed. The measured values of the amount of alanine before and after pH shift are shown in Table 6.

Example 7

In the same manner as in Example 1, except that the yeast to be cultured is Saccharomyces cerevisiae ABS 3 strain, a yeast was cultured, and extraction of an extract from a yeast culture fluid and alanine analysis were performed. The measured values of the amount of alanine before and after pH shift are shown in Table 6.

Example 8

In the same manner as in Example 1, except that the yeast to be cultured was Camellia (bread yeast), a yeast was cultured, and extraction of an extract from a yeast culture fluid and alanine analysis were performed. The measured values of the amount of alanine before and after pH shift are shown in Table 6.

Example 9

In the same manner as in Example 1, except that the yeast to be cultured was Candida utilis ABC1 strain, a yeast was cultured, and extraction of an extract from a yeast culture fluid and alanine analysis were performed. The measured values of the amount of alanine before and after pH shift are shown in Table 6.

Example 10

In the same manner as in Example 1, except that the yeast to be cultured was Candida utilis ABC2 strain, a yeast was cultured, and extraction of an extract from a yeast culture fluid and alanine analysis were performed. The measured values of the amount of alanine before and after pH shift are shown in Table 6.

Example 11

In the same manner as in Example 1, except that the yeast to be cultured was Candida utilis ABC3 strain, a yeast was cultured, and extraction of an extract from a yeast culture fluid and alanine analysis were performed. The measured values of the amount of alanine before and after pH shift are shown in Table 6.

Table 6

| Bacterial strains | Set pH | Before pH shift | After pH shift |
|---|---|---|---|
| | | Alanine (% by weight in dry yeast cells) | Alanine (% by weight in dry yeast cells) |
| Saccharomyces cerevisiae ABS1 | 8.0 | 1.13 | 1.68 |
| Saccharomyces cerevisiae ABS2 | 8.0 | 0.37 | 0.58 |
| Saccharomyces cerevisiae ABS3 | 8.0 | 0.44 | 1.92 |
| Camellia (Baker's yeast) | 8.0 | 0.27 | 2.71 |
| Candida utilis ABC1 | 8.0 | 0.26 | 1.01 |
| Candida utilis ABC2 | 8.0 | 0.15 | 1.16 |
| Candida utilis ABC3 | 8.0 | 0.14 | 2.77 |

As is apparent from the results of Table 6, a phenomenon of increase in the amount of alanine was confirmed by pH shift, also in other strains of Saccharomyces cerevisiae, and the yeast of other genus which are tested in Examples. In addition, the amounts (% by weight) of alanine in the extract before and after pH shift of Examples 5 to 11 are as follows.

Experiment No.: before shift -> after shift)
(Example 5: 4.05 -> 10.03),
(Example 6: 1.17 -> 5.99),
(Example 7: 1.55 -> 6.03),
(Example 8: 0.96 -> 4.20),
(Example 9: 1.05 -> 10.10),

(Example 10: 0.54 -> 3.98),
(Example 11: 0.61 -> 8.46).

Example 12

In the same as that of Example 1, except that yeast to be cultured was Saccharomyces cerevisiae ABS4 strain, and the set pH of pH shift was in units of 0.5 between 7.0 to 9.5, a yeast was cultured and extraction of an extract from yeast culture and alanine analysis were performed. The measured values of the amount of alanine before and after pH shift are shown in Table 7.

Table 7

| Saccharomyces cerevisiae | Set pH | Before pH shift | After pH shift |
| --- | --- | --- | --- |
| | | Alanine (% by weight in dry yeast cells) | Alanine (% by weight in dry yeast cells) |
| ABS4 | 7.0 | 3.34 | 5.61 |
| ABS4 | 7.5 | 3.43 | 5.46 |
| ABS4 | 8.0 | 3.60 | 9.43 |
| ABS4 | 8.5 | 2.95 | 11.30 |
| ABS4 | 9.0 | 4.39 | 6.84 |
| ABS4 | 9.5 | 3.28 | 4.74 |

As is apparent from the results of Table 7, an increase in the amount of alanine to 4.0 to 12.0% by weight was seen in the case of all set pHs and also in Saccharomyces cerevisiae ABS4 strain.

[Industrial Applicability]

According to the method for producing an alanine-rich yeast of the present invention, a yeast containing alanine maintained in a high concentration in cells can be obtained and therefore the present invention can be utilized in the field of food industry such as production of a yeast extract.

**Claims**

1. A method for producing an alanine-rich yeast, comprising: liquid-culturing a yeast in a stationary phase of proliferation under the condition that a pH of a liquid medium is equal to or higher than 7.5 and lower than 11.

2. The method for producing an alanine-rich yeast according to claim 1, wherein the liquid-culturing further comprises adjusting the pH of the liquid medium of the yeast in a stationary phase of proliferation to be equal to or higher than 7.5 and lower than 11, and culturing the yeast in the above pH range.

3. The method for producing an alanine-rich yeast according to claim 1 or 2, wherein the yeast is Saccharomyces cerevisiae or Candida utilis.

4. An alanine-rich yeast obtained by the method for producing an alanine-rich yeast according to any one of claims 1 to 3.

5. An alanine-rich yeast extract extracted from the alanine-rich yeast according to claim 4.

6. A seasoning composition comprising the alanine-rich yeast extract according to claim 5.

7. An alanine-containing food and drink, comprising the alanine-rich yeast according to claim 4, the alanine-rich yeast extract according to claim 5 or the seasoning composition according to claim 6.

FIG. 1

FIG. 2

FIG. 3

pH BEHAVIOR

<table>
<tr><td colspan="2" align="center">**INTERNATIONAL SEARCH REPORT**</td><td colspan="2">International application No.<br>PCT/JP2009/006165</td></tr>
</table>

A.  CLASSIFICATION OF SUBJECT MATTER
*C12N1/16*(2006.01)i, *A23L1/221*(2006.01)i, *A23L1/28*(2006.01)i, *A23L1/39* (2006.01)i

According to International Patent Classification (IPC) or to both national classification and IPC

B.  FIELDS SEARCHED

Minimum documentation searched (classification system followed by classification symbols)
C12N1/16, A23L1/221, A23L1/28, A23L1/39

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
JSTPlus/JMEDPlus/JST7580(JDreamII), G-Search, Shokuhin Kanren Bunken Joho (Shoku Net), WPI/FOODLINE/Foods Adlibra/Food Sci. & Tech. Abs(DIALOG)

C.  DOCUMENTS CONSIDERED TO BE RELEVANT

| Category* | Citation of document, with indication, where appropriate, of the relevant passages | Relevant to claim No. |
|---|---|---|
| Y | JP 39-16342 B1  (Ajinomoto Co., Inc.),<br>11 August 1964 (11.08.1964),<br>example 2<br>(Family: none) | 1-7 |
| Y | JP 54-31076 B1  (Ajinomoto Co., Inc.),<br>04 October 1979 (04.10.1979),<br>claim 1; page 2, column 3, lines 40 to 44<br>(Family: none) | 1-7 |
| Y | JP 60-133892 A  (Genex Corp.),<br>17 July 1985 (17.07.1985),<br>claim 13<br>& US 4584269 A          & EP 143560 A2 | 1-7 |

☒  Further documents are listed in the continuation of Box C. ☐  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

Date of the actual completion of the international search
21 December, 2009 (21.12.09)

Date of mailing of the international search report
28 December, 2009 (28.12.09)

Name and mailing address of the ISA/
Japanese Patent Office

Authorized officer

Facsimile No.

Telephone No.

Form PCT/ISA/210 (second sheet) (April 2007)

**INTERNATIONAL SEARCH REPORT**

| International application No. |
| --- |
| PCT/JP2009/006165 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 63-123390 A  (Idemitsu Kosan Co., Ltd.),<br>27 May 1988 (27.05.1988),<br>page 2, lower right column, lines 1 to 9<br>(Family: none) | 1-7 |
| Y | JP 09-313169 A  (Ajinomoto Co., Inc.),<br>09 December 1997 (09.12.1997),<br>claims; paragraph [0023]<br>(Family: none) | 1-7 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007049989 A

- JP 3519572 B